(19) Europäisches Patentamt
European Patent Office
Office européen des brevets

(11) Veröffentlichungsnummer: **0 212 354**
**A2**

(12) **EUROPÄISCHE PATENTANMELDUNG**

(21) Anmeldenummer: **86110413.1**

(22) Anmeldetag: **28.07.86**

(51) Int. Cl.⁴: **C 07 D 231/38**, C 07 D 231/16, C 07 D 401/04, A 01 N 43/56

(30) Priorität: **08.08.85 DE 3528477**

(43) Veröffentlichungstag der Anmeldung: **04.03.87 Patentblatt 87/10**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Gehring, Reinhold, Dr., Dasnöckel 49, D-5600 Wuppertal 11 (DE)**
Erfinder: **Schallner, Otto, Dr., Noldeweg 22, D-4019 Monheim (DE)**
Erfinder: **Stetter, Jörg, Dr., Gellertweg 4, D-5600 Wuppertal (DE)**
Erfinder: **Lürssen, Klaus, Dr., August-Kierspel-Strasse 151, D-5060 Bergisch-Gladbach 2 (DE)**
Erfinder: **Santel, Hans-Joachim, Dr., Gruenstrasse 9a, D-5090 Leverkusen (DE)**
Erfinder: **Schmidt, Robert R., Dr., Im Waldwinkel 110, D-5060 Bergisch-Gladbach 2 (DE)**

(54) **1- Aryl-pyrazole.**

(57) Die Erfindung betrifft neue 1-Aryl-pyrazole der allgemeinen Formel (I)

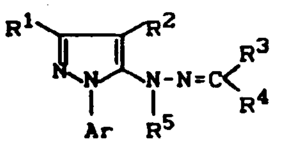

(I)

in welcher
$R^1$ für Wasserstoff oder Alkyl steht,
$R^2$ für Cyano oder Nitro steht,
$R^3$ und $R^4$ unabhängig voneinander jeweils für Wasserstoff, Alkyl, Alkoxyalkyl, Alkylthioalkyl, Halogenalkyl oder für gegebenenfalls substituiertes Aryl stehen, oder
$R^3$ und $R^4$ gemeinsam für einen zweifach verknüpften Alkylenrest stehen,
$R^5$ für Wasserstoff oder Alkyl steht
und
Ar für gegebenenfalls substituiertes Phenyl oder für gegebenenfalls substituiertes Pyridyl steht,
mehrere Verfahren zu ihrer Herstellung und ihre Verwendung als Herbizide und Pflanzenwachstumsregulatoren.

EP 0 212 354 A2

BAYER AKTIENGESELLSCHAFT
Konzernverwaltung RP
Patentabteilung

5090 Leverkusen, Bayerwerk
KM/ABc
(Ib)

# 1-Aryl-pyrazole

Die Erfindung betrifft neue 1-Aryl-pyrazole, mehrere Verfahren zu ihrer Herstellung und ihre Verwendung als Herbizide und Pflanzenwachstumsregulatoren.

Es ist bereits bekannt, daß bestimmte 1-Aryl-pyrazole wie beispielsweise das 4-Cyano-5-propionamido-1-(2,4,6-trichlorphenyl)-pyrazol herbizide Eigenschaften besitzen (vgl. z.B. DE-OS 3 226 513).

Die herbizide Wirksamkeit dieser vorbekannten Verbindungen gegenüber Schadpflanzen ist jedoch ebenso wie ihre Verträglichkeit gegenüber wichtigen Kulturpflanzen nicht immer in allen Anwendungsbereichen völlig zufriedenstellend.

Über eine Wirksamkeit als Wachstumsregulatoren ist im Zusammenhang mit diesen vorbekannten 1-Aryl-pyrazolen nichts bekannt.

Es wurden nun neue 1-Aryl-pyrazole der allgemeinen Formel (I)

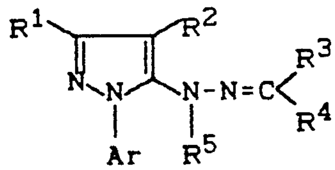

(I)

in welcher

$R^1$ für Wasserstoff oder Alkyl steht,

$R^2$ für Cyano oder Nitro steht,

$R^3$ und $R^4$ unabhängig voneinander jeweils für Wasserstoff, Alkyl, Alkoxyalkyl, Alkylthioalkyl, Halogenalkyl oder für gegebenenfalls substituiertes Aryl stehen, oder

$R^3$ und $R^4$ gemeinsam für einen zweifach verknüpften Alkylenrest stehen,

$R^5$ für Wasserstoff oder Alkyl steht

und

Ar für gegebenenfalls substituiertes Phenyl oder für gegebenenfalls substituiertes Pyridyl steht,

gefunden.

Weiterhin wurde gefunden, daß man die neuen 1-Aryl-pyrazole der allgemeinen Formel (I),

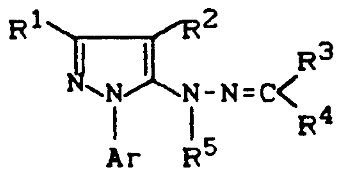

(I)

in welcher

$R^1$ für Wasserstoff oder Alkyl steht,

$R^2$ für Cyano oder Nitro steht,

$R^3$ und $R^4$ unabhängig voneinander jeweils für Wasserstoff, Alkyl, Alkoxyalkyl, Alkylthioalkyl, Halogenalkyl oder für gegebenenfalls substituiertes Aryl stehen, oder

$R^3$ und $R^4$ gemeinsam für einen zweifach verknüpften Alkylenrest stehen,

$R^5$ für Wasserstoff oder Alkyl steht

und

Ar für gegebenenfalls substituiertes Phenyl oder für gegebenenfalls substituiertes Pyridyl steht,

erhält, wenn man

(a) 1-Aryl-5-hydrazino-pyrazole der Formel (II),

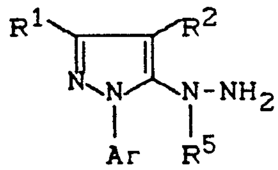

(II)

in welcher

$R^1$, $R^2$, $R^5$ und Ar die oben angegebene Bedeutung haben,

mit Aldehyden oder Ketonen der Formel (III),

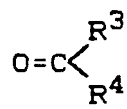

(III)

in welcher

$R^3$ und $R^4$ die oben angegebene Bedeutung haben,

gegebenenfalls in Gegenwart eines Verdünnungsmittels umsetzt, oder wenn man

(b) die nach Verfahren (a) erhältlichen 1-Aryl-pyrazole der Formel (Ia),

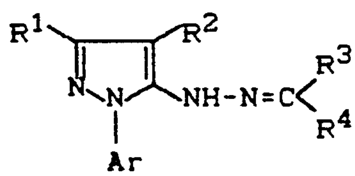

(Ia)

in welcher

$R^1$, $R^2$, $R^3$, $R^4$ und Ar die oben angegebene Bedeutung haben,

mit Alkylierungsmitteln der Formel (IV),

$$R^{5-1}\text{-A} \quad \text{(IV)}$$

in welcher

$R^{5-1}$ für Alkyl steht und

A für eine elektronenanziehende Abgangsgruppe steht,

gegebenenfalls in Gegenwart eines Verdünnungsmittels und gegebenenfalls in Gegenwart eines Säurebindemittels oder basischen Katalysators umsetzt.

Schließlich wurde gefunden, daß die neuen 1-Aryl-pyrazole der Formel (I) herbizide insbesondere auch selektivherbizide Eigenschaften und darüberhinaus auch pflanzenwachstumsregulatorische Eigenschaften besitzen.

Überraschenderweise zeigen die erfindungsgemäßen 1-Arylpyrazole der allgemeinen Formel (I) bei vergleichbar guter herbizider Wirksamkeit gegenüber wichtigen Problemunkräutern eine erheblich verbesserte Verträglich-

keit gegenüber wichtigen Nutzpflanzen als die aus dem Stand der Technik bekannten 1-Aryl-pyrazole wie beispielsweise das 4-Cyano-5-propionamido-1-(2,4,6-trichlorphenyl)-pyrazol, welche chemisch und wirkungsmäßig naheliegende Verbindungen sind, und darüberhinaus völlig unerwarteterweise zusätzlich eine pflanzenwachstumsregulatorische Wirksamkeit.

Die erfindungsgemäßen 1-Aryl-pyrazole sind durch die Formel (I) allgemein definiert. Bevorzugt sind Verbindungen der Formel (I), bei welchen

$R^1$ für Wasserstoff oder für geradkettiges oder verzweigtes Alkyl mit 1 bis 4 Kohlenstoffatomen steht,

$R^2$ für Cyano oder Nitro steht,

$R^3$ und $R^4$ unabhängig voneinander jeweils für Wasserstoff, für jeweils geradkettiges oder verzweigtes Alkyl, Alkoxyalkyl, Alkylthioalkyl oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen, oder für gegebenenfalls einfach oder mehrfach, gleich oder verschieden substituiertes Phenyl stehen, wobei als Substituenten jeweils in Frage kommen:
Halogen, Cyano, Nitro oder jeweils geradkettiges oder verzweigtes Alkyl, Alkoxy, Alkylthio oder Halogenalkyl mit jeweils 1 bis 4 Kohlenstoffatomen und im Fall des Halogenalkyl mit 1 bis 9 gleichen oder verschiedenen Halogenatomen, oder

$R^3$ und $R^4$ gemeinsam für einen geradkettigen oder verzweigten, zweifach verknüpften Alkylenrest mit 3 bis 12 Kohlenstoffatomen stehen,

$R^5$ für Wasserstoff oder für geradkettiges oder verzweigtes Alkyl mit 1 bis 4 Kohlenstoffatomen steht und

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils in Frage kommen:
Cyano, Nitro, Halogen, jeweils geradkettiges oder verzweigtes Alkyl, Alkoxy oder Alkoxycarbonyl mit jeweils 1 bis 4 Kohlenstoffatomen im Alkylteil, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^6$

wobei

$R^6$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen

oder verschiedenen Halogenatomen steht und

m für eine Zahl 0, 1 oder 2 steht.

Besonders bevorzugt sind 1-Aryl-pyrazole der Formel (I), bei welchen

$R^1$ für Wasserstoff, Methyl, Ethyl, n- oder i-Propyl, sowie n-, i-, s- oder t-Butyl steht,

$R^2$ für Cyano oder Nitro steht,

$R^3$ und $R^4$ unabhängig voneinander jeweils für Wasserstoff, Methyl, Ethyl, n- oder i-Propyl, n-, i-, s- oder t-Butyl, Methoxymethyl, Methylthiomethyl, Chlormethyl, Dichlormethyl, Trichlormethyl, Trifluormethyl, Chlorethyl, Bromethyl, Trichlorethyl, Trifluorethyl oder für gegebenenfalls ein- bis dreifach, gleich oder verschieden substituiertes Phenyl stehen, wobei als Substituenten jeweils in Frage kommen: Fluor, Chlor, Brom, Cyano, Nitro, Methyl, Ethyl, Methoxy, Methylthio oder Trifluormethyl, oder

$R^3$ und $R^4$ gemeinsam für einen geradkettigen, zweifach verknüpften Alkylenrest mit 4 bis 7 Kohlenstoffatomen stehen,

$R^5$ für Wasserstoff, Methyl oder Ethyl steht und

Ar für gegebenenfalls ein- bis fünffach, gleich oder verschieden substituiertes Phenyl oder für jeweils gegebenenfalls ein- bis vierfach, gleich oder verschieden substituiertes 2-Pyridyl oder 4-Pyridyl steht, wobei als Substituenten jeweils in Frage kommen: Cyano, Nitro, Fluor, Chlor, Brom, Iod, Methyl, Ethyl, n- oder i-Propyl, n-, i-, s- oder t-Butyl, Methoxy, Ethoxy, Methoxycarbonyl, Ethoxycarbonyl, Trifluormethyl, Trichlormethyl, Difluorchlormethyl, Difluorchlorethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^6$, wobei

$R^6$ für Amino, Methylamino, Ethylamino, Dimethylamino, Diethylamino, Methyl, Ethyl, Fluordichlormethyl, Difluormethyl, Tetrafluorethyl, Trifluorchlorethyl oder Trifluormethyl steht und

m für eine Zahl 0, 1 oder 2 steht.

Im einzelnen seien außer den bei den Herstellungsbeispielen genannten Verbindungen die folgenden 1-Arylpyrazole der allgemeinen Formel (I) genannt:

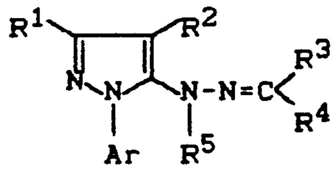

(I)

Tabelle 1:

| $R^1$ | $R^2$ | $R^3$ | $R^4$ | $R^5$ | Ar |
|---|---|---|---|---|---|
| H | $NO_2$ | $CH_3$ | $C_2H_5$ | H | 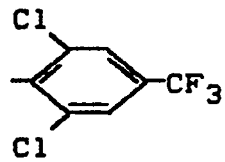 |
| H | $NO_2$ | $CH_3$ | $i-C_3H_7$ | H | 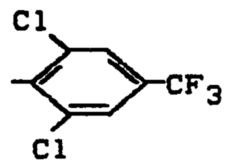 |
| H | $NO_2$ | $CH_3$ | $i-C_4H_9$ | H | 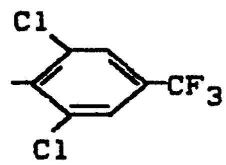 |
| H | $NO_2$ | $CH_3$ | 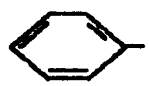 | H | 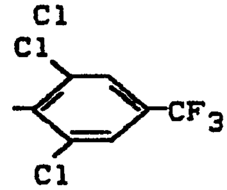 |
| H | $NO_2$ | $CH_3$ | $CH_3$ | $CH_3$ | 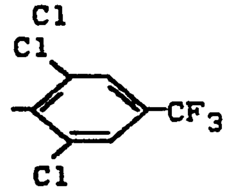 |
| H | $NO_2$ | $CH_3$ | $CH_2-OCH_3$ | H | 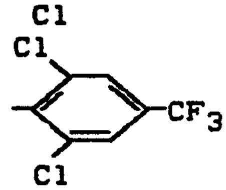 |
| H | $NO_2$ | $CH_3$ | $CH_2Cl$ | H | 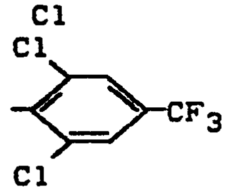 |

Tabelle 1 (Fortsetzung)

| $R^1$ | $R^2$ | $R^3$ | $R^4$ | $R^5$ | Ar |
|---|---|---|---|---|---|
| H | $NO_2$ | $CH_3$ | $CH_2-SCH_3$ | H | 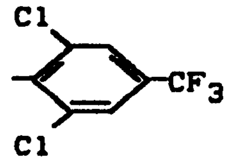 |
| H | $NO_2$ | $CH_3$ | $CH_3$ | H | 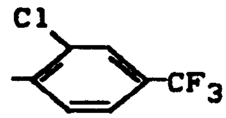 |
| H | CN | $CH_3$ | $C_2H_5$ | H | 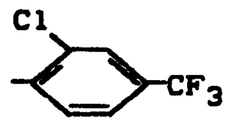 |
| H | CN | $CH_3$ | $i-C_3H_7$ | H | 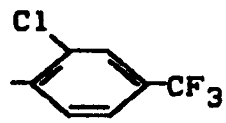 |
| H | CN | $CH_3$ | $i-C_4H_9$ | H | 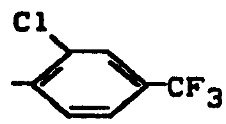 |
| H | CN | $CH_3$ | 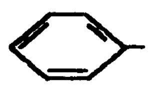 | H | 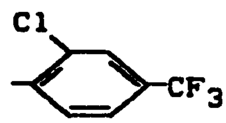 |
| H | CN | $CH_3$ | $CH_2-OCH_3$ | H | 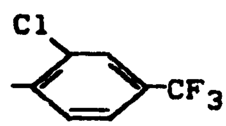 |
| H | CN | $CH_3$ | $CH_2Cl$ | H | 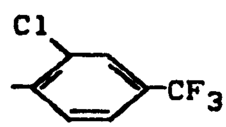 |

**Tabelle 1** (Fortsetzung):

| $R^1$ | $R^2$ | $R^3$ | $R^4$ | $R^5$ | Ar |
|---|---|---|---|---|---|
| H | CN | $CH_3$ | $CH_2-SCH_3$ | H | 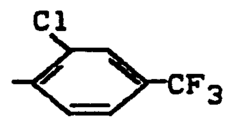 |
| H | CN | $CH_3$ | 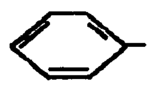 | H | 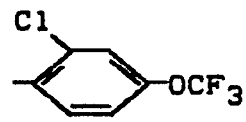 |
| H | CN | H | 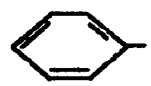 | H | 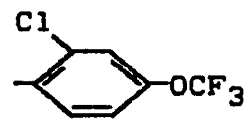 |

Verwendet man beispielsweise 4-Cyano-5-hydrazino-1-(2,4-dichlorphenyl)-pyrazol und Aceton als Ausgangsstoffe, so läßt sich der Reaktionsablauf des erfindungsgemäßen Verfahrens (a) durch das folgende Formelschema darstellen:

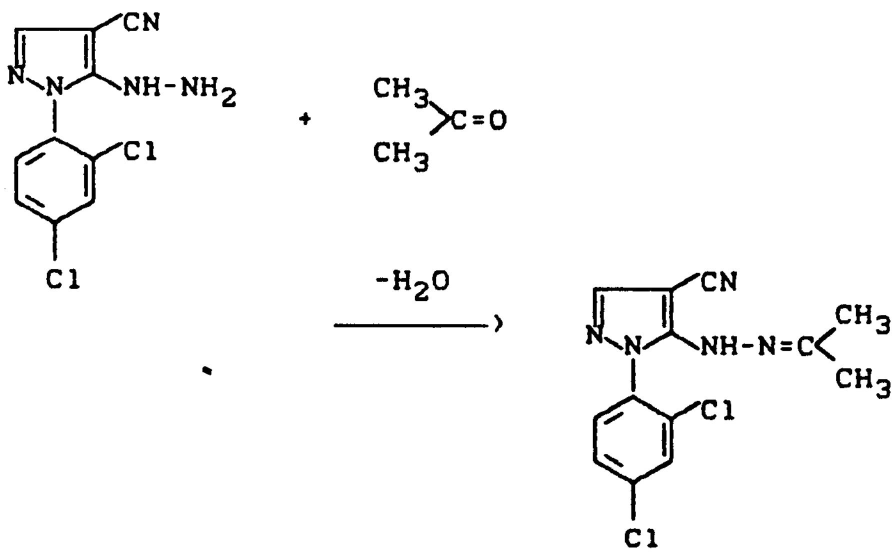

Verwendet man beispielsweise 4-Cyano-5-(2-propylidenimino)-amino-1-(2,6-dichlor-4-trifluormethyl-phenyl)-pyrazol und Methyliodid als Ausgangsstoffe, so läßt sich der Reaktionsablauf des erfindungsgemäßen Verfahrens (b) durch das folgende Formelschema darstellen:

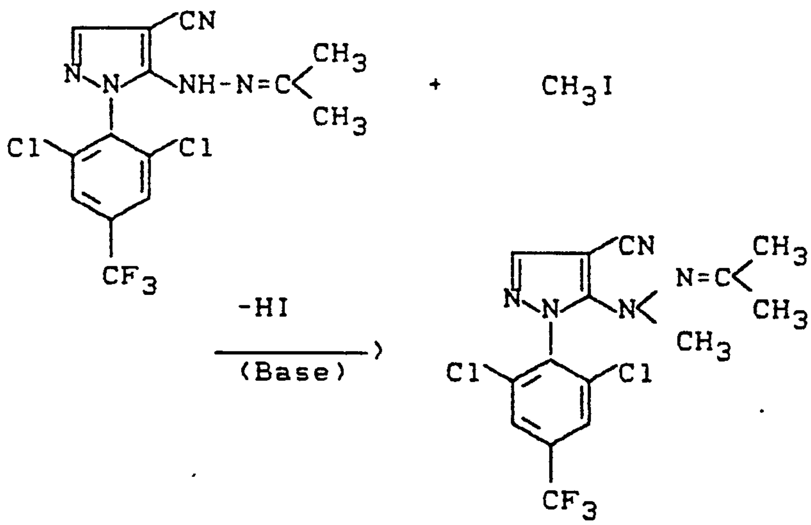


Die zur Durchführung des erfindungsgemäßen Verfahrens (a) als Ausgangsstoffe benötigten 1-Aryl-5-hydrazino-pyrazole sind durch die Formel (II) allgemein definiert. In dieser Formel (II) stehen $R^1$, $R^2$, $R^5$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Aryl-5-hydrazino-pyrazole der Formel (II) sind teilweise bekannt (vgl. z.B. J. Heterocycl. Chem. <u>20</u>, 277-279 [1983]), teilweise sind sie Gegenstand einer eigenen parallelen Patentanmeldung. Man erhält sie beispielsweise, wenn man 1-Aryl-5-halogen-pyrazole der Formel (V),

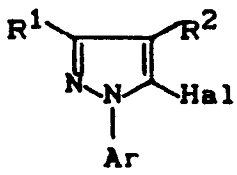

(V)

in welcher

$R^1$, $R^2$ und Ar die oben angegebene Bedeutung haben und

Hal für Halogen steht,

mit Hydrazin-Derivaten der Formel (VI),

$H_2N-NH-R^5$ (VI)

in welcher

$R^5$ die oben angegebene Bedeutung hat,

gegebenenfalls in Gegenwart eines Verdünnungsmittels wie beispielsweise Dioxan bei Temperaturen zwischen 20°C und 150°C umsetzt.

Die 1-Aryl-5-halogen-pyrazole der Formel (V) sind noch nicht bekannt. Sie sind jedoch zum größten Teil Gegenstand eigener vorgängiger Patentanmeldungen (vgl. DE-P 3 501 323 vom 17.01.1985 und DE-P 35 20 329 vom 07.06.1985).

Man erhält sie beispielsweise, wenn man 5-Amino-1-aryl-pyrazole der Formel (VII),

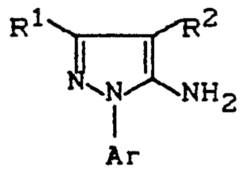

(VII)

in welcher

$R^1$, $R^2$ und Ar die oben angegebene Bedeutung haben,

mit Nitritverbindungen der Formel (VIII)

R-O-N=O (VIII)

in welcher

R für Wasserstoff, Alkyl oder für ein Alkalimetallkation steht,

in Gegenwart einer Halogenwasserstoffsäure wie beispielsweise Chlorwasserstoffsäure oder Bromwasserstoffsäure oder in Gegenwart eines Haloforms wie beispielsweise Chloroform oder Bromoform bei Temperaturen zwischen -20°C und +80°C in üblicher Art und Weise diazotiert (vgl. z.B. "Organikum" 15.Auflage VEB Deutscher Verlag der Wissenschaften, Berlin 1981, S. 652 ff; J. Chem. Soc. C, <u>1966</u>, 1249 oder Rev. Latinoam. Quim. <u>13</u>, 100-102 [1982]).

Die 5-Amino-1-aryl-pyrazole der Formel (VII) sind teilweise bekannt (vgl. z.B. EP 26 034, EP 53 678 oder EP 34 945 sowie DE-OS 3 226 496, DE-OS 3 408 727 oder DE-OS 3 420 985), teilweise sind sie Gegenstand eigener noch nicht vorveröffentlichter Patentanmeldungen (vgl. DE-P 3 402 308 vom 24.01.1984; vgl. auch DE-P 35 20 330 vom 07.06.1985 und DE-P 35 20 327 vom 07.06.85).

Man erhält sie beispielsweise, wenn man Aryl-hydrazine der Formel (IX),

$$Ar-NH-NH_2 \qquad (IX)$$

in welcher

Ar die oben angegebene Bedeutung hat,

(α) mit Acrylnitril-Derivaten der Formel (X),

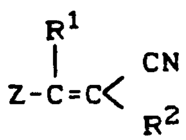

(X)

in welcher

$R^1$ und $R^2$ die oben angegebene Bedeutung haben und

Z für Halogen, Hydroxy, Alkoxy oder Dialkylamino steht, oder

(β) mit 2-Halogenacrylnitrilen der Formel (XI),

$$R^1\text{-CH=C}\Big\langle\begin{matrix}CN\\ Hal^1\end{matrix} \qquad (XI)$$

in welcher

$R^1$ die oben angegebene Bedeutung hat und

$Hal^1$ für Halogen, insbesondere für Chlor oder Brom steht,

entweder zunächst in einer 1. Stufe, gegebenenfalls in Gegenwart eines Verdünnungsmittels wie beispielsweise Eisessig oder Ethanol sowie gegebenenfalls in Gegenwart eines Reaktionshilfsmittels wie beispielsweise Natriumacetat bei Temperaturen zwischen -20°C und +20°C umsetzt zu den Arylhydrazin-Derivaten der Formel (XII),

$$\text{Ar-NH-NH-}\overset{\displaystyle R^1}{\overset{|}{C}}\text{=C}\Big\langle\begin{matrix}CN\\ R^{2-1}\end{matrix} \qquad (XII)$$

in welcher

Ar die oben angegebene Bedeutung hat und

$R^{2-1}$ für Halogen, Cyano oder Nitro steht

und diese in einer 2. Stufe, gegebenenfalls in Gegenwart eines Verdünnungsmittels wie beispielsweise Ethylenglykolmonoethylether und gegebenenfalls in Gegenwart eines sauren Katalysators wie beispielsweise Schwefelsäure oder Phosphorsäure bei Temperaturen zwischen +50°C und +150°C cyclisiert,

oder direkt in einem Reaktionsschritt, ohne Isolierung der Zwischenstufe der Formel (XII) gegebenenfalls in Gegenwart eines Verdünnungsmittels beispielsweise Ethylenglykolmonoethylether oder Ethanol bei Temperaturen zwischen +50°C und +150°C cyclisiert und gegebenenfalls die nach der Variante (β) erhältlichen in 4-Stellung unsubstituierten 5-Aminopyrazole der Formel (XIII),

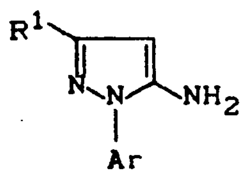

(XIII)

in welcher

$R^1$ und Ar die oben angegebenen Bedeutungen haben,

in einer Folgereaktion mit einem Nitrierungsmittel wie beispielsweise Salpetersäure gegebenenfalls in Gegenwart eines Verdünnungsmittels wie beispielsweise Eisessig und gegebenenfalls in Gegenwart eines Reaktionshilfsmittels wie beispielsweise Acetanhy-

drid bei Temperaturen zwischen -20°C und +50°C nitriert.

Dabei kann es gegebenenfalls von Vorteil sein, vor der Nitrierungsreaktion die Aminogruppe in der 5-Position des Pyrazolringes mit Hilfe üblicher Schutzgruppentechnik, beispielsweise durch Acylierung, zu schützen und die Aminoschutzgruppe nach erfolgter Nitrierung ebenfalls in üblicher Art und Weise beispielsweise durch Verseifung mit wässriger oder alkoholischer Base wieder abzuspalten.

Die Arylhydrazine der Formel (IX) sind bekannt (vgl. z.B. US-PS 4 127 575; US-PS 3 609 158; DE-OS 2 558 399; J. Chem. Soc. C, 1971, 167-174) oder können nach bekannten Verfahren in einfacher analoger Weise erhalten werden (vgl. z.B Houben-Weyl "Methoden der organischen Chemie" Band X/2, S. 203, Thieme Verlag Stuttgart, 1967), indem man beispielsweise die bekannten Aniline bzw. Pyridylamine der Formel (XIV),

$$Ar\text{-}NH_2 \qquad (XIV)$$

in welcher

Ar die oben angegebene Bedeutung hat,

mit Natriumnitrit in Gegenwart einer Säure wie beispielsweise Schwefelsäure, und dann mit Zinn-II-chlorid ebenfalls in Gegenwart einer Säure wie beispielsweise Salzsäure bei Temperaturen zwischen -20°C und +80°C umsetzt oder wenn man Halogenaromaten der Formel (XV),

Ar-$Hal^2$ (XV)

in welcher

Ar die oben angegebene Bedeutung hat und

$Hal^2$ für Halogen, insbesondere für Fluor, Chlor oder Brom steht,

mit Hydrazinhydrat gegebenenfalls in Gegenwart eines Verdünnungsmittels wie beispielsweise Pyridin oder Dioxan bei Temperaturen zwischen 0° und 150°C umsetzt.

Die Hydrazin-Derivate der Formel (VI), die Nitritverbindungen der Formel (VIII), die Acrylnitril-Derivate der Formel (X), die 2-Halogenacrylnitrile der Formel (XI), die Aniline und Pyridylamine der Formel (XIV) und die Halogenaromaten der Formel (XV) sind allgemein bekannte Verbindungen der organischen Chemie.

Die zur Durchführung des erfindungsgemäßen Verfahrens (a) weiterhin als Ausgangsstoffe benötigten Aldehyde und Ketone sind durch die Formel (III) allgemein definiert. In dieser Formel (III) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Aldehyde und Ketone der Formel (III) sind allgemein bekannte Verbindungen der organischen Chemie.

Die zur Durchführung des erfindungsgemäßen Verfahrens (b) als Ausgangsstoffe benötigten 1-Aryl-pyrazole sind durch die Formel (Ia) allgemein definiert. In dieser Formel (Ia) stehen $R^1$, $R^2$, $R^3$, $R^4$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Aryl-pyrazole der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Ausgangsstoffe benötigten Alkylierungsmittel sind durch die Formel (IV) allgemein definiert. In dieser Formel (IV) steht $R^{5-1}$ vorzugsweise für geradkettiges oder verzweigtes Alkyl mit 1 bis 4 Kohlenstoffatomen, insbesondere für Methyl oder Ethyl.
A steht vorzugsweise für Chlor, Brom oder Iod, für p-Toluolsulfonyloxy oder Methoxysulfonyloxy.

Die Alkylierungsmittel der Formel (IV) sind allgemein bekannte Verbindungen der organischen Chemie.

Als Verdünnugsmittel zur Durchführung des erfindungsgemäßen Verfahrens (a) kommen inerte organische Verdünnungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Verwendet man als Reaktionspartner Ketone oder Aldehyde der Formel (III) in flüssiger Form, so können diese mit besonderem Vorzug in entsprechendem Überschuß gleichzeitig als Verdünnungsmittel verwendet werden.

Die Reaktionstemperaturen können bei der Durchführung des erfindungsgemäßen Verfahrens (a) in einem größeren Bereich variiert werden. Im allgemeinen arbeitet man bei Temperaturen zwischen 20°C und 150°C, vorzugsweise bei Temperaturen zwischen 30°C und 100°C.

Zur Durchführung des erfindungsgemäßen Verfahrens (a) setzt man pro Mol an 1-Aryl-5-hydrazino-pyrazol der Formel (II) im allgemeinen 1,0 bis 30,0 Mol, vorzugsweise 1.0 bis 10,0 Mol an Keton oder Aldehyd der Formel (III) ein. Die Reationsdurchführung, Aufarbeitung und Isolierung der Reaktionsprodukte der Formel (I) erfolgt nach allgemein üblichen Methoden.

Als Verdünnungsmittel zur Durchführung des erfindungsgemäßen Verfahrens (b) kommen ebenfalls inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (b) wird gegebenenfalls in Gegenwart einer als Katalysator und/oder Säurebindemittel wirkenden Base durchgeführt.

Als solche kommen alle üblichen anorganischen oder organischen Basen in Frage. Hierzu gehören beispielsweise Alkalimetallhydroxide, -hydride oder -alkoholate wie Natriumhydroxid oder Kaliumhydroxid, Natriumhydrid, Natriummethylat oder Kalium-t-butylat, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen(DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +120°C, vorzugsweise bei Temperaturen zwischen 0°C und 100°C.

Zur Durchführung des erfindungsgemäßen Verfahrens (b) setzt man pro Mol an 1-Aryl-pyrazol der Formel (Ia) im allgemeinen 1,0 bis 30,0 Mol, vorzugsweise 1,0 bis 15,0 Mol an Alkylierungsmittel der Formel (IV) und gegebenenfalls 0,1 bis 5,0 Mol, vorzugsweise 0,5 bis 1,5 Mol an Base ein. Die Reaktionsdurchführung, Aufarbeitung und Isolierung der Reaktionsprodukte der Formel (I) erfolgt nach üblichen Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono- und dikotyler Unkräuter, vorzugsweise in monokotylen Kulturen wie beispielsweise Weizen einsetzen.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch

wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung

sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, da z.B. bei Tabak, Tomaten oder Kaffe e eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Aerosole, Suspensions-Emulsions-Konzentrate, Wirkstoff-imprägnierte Natur- und synthetische Stoffe, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Als feste Trägerstoffe kommen in Frage: Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie

Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4-(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-trifluormethyl-phenyl)-harnstoff; N,N-Dimethyl-N'-(3-chlor-4-methylphenyl)-harnstoff; N,N-Dimethyl-N'-(4-isopropylphenyl)-harnstoff; 4-Amino-6-t-butyl-3-ethylthio-1,2,4-triazin-5-(4H)-on; 2,4-Dichlorphenoxyessigsäure; 2,4-Dichlorphenoxy-propionsäure; (2-Methyl-4-chlorphenoxy)-essigsäure; (4-Chlor-2-methylphenoxy)-propionsäure; Chloressigsäure-N-(methoxymethyl)-2,6-diethylanilid; 2-Ethyl-6-methyl-N-(1-methyl-2-methoxyethyl)-chloracetanilid; 2,6-Dinitro-4-trifluormethyl-N,N-dipropylanilin; 2-[4-(3,5-Dichlorpyrid-2-yloxy)-phenoxy]-propionsäure-2-(ben-zyloxyethylester), -(trimethylsilylmethylester) oder -(2,2-diethoxyethylester); 2-[1-(Ethoxyamino)-butyliden]-5-(2-ethylthiopropyl)-1,3-cylcohexandion; Methyl-5-(2,4-dichlorphenoxy)-2-nitrobenzoat; 3,5-Diiod-4-hydroxybenzo-nitril; 3-Isopropyl-2,1,3-benzothiadiazin-4-on-2,2-dioxid; 2-Chlor-N-{[(4-methoxy-6-methyl-1,3,5-triazin-2-yl)-amino]-carbonyl}-benzolsulfonamid; 4-Ethylamino-2-t-butylamino-6-methylthio-s-triazin; 2-{4-[(3-Chlor-5-(trifluormethyl)-2-pyridyl)-oxy]-phenoxy}-propansäure bzw.-propansäureethylester; 5-(2-Chlor-4-trifluormethyl-phenoxy)-2-nitro-benzoesäure; N-(1-Ethylpropyl)-3,4-dimethyl-2,6-dinitroanilin; N-Methyl-2-(1,3-benzthiazol-2-yloxy)-acetanilid; N,N-Diisopropyl-S-(2,3,3-trichlor-allyl)-thiolcarbamat; sowie 2-[5-Methyl-(1-methylethyl)-4-oxo-2-imidazolin-2-yl]-3-chinolincarbonsäure sind gegebenenfalls von Vorteil. Einige Mischungen zeigen überraschenderweise auch synergistische Wirkung.

Auch eine Mischung mit anderen bekannten Wirkstoffen wie Fungiziden, Insektiziden, Akariziden, Nematiziden, Schutzstoffen gegen Vogelfraß, Pflanzennährstoffen und Bodenstrukturverbesserern ist möglich.

Die Wirkstoffe können als solche, in Form ihrer Formulierungen oder den daraus durch weiteres Verdünnen bereiteten Anwendungsformen wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Bei der Verwendung als Wachstumsregulatoren können die erfindungsgemäßen Wirkstoffe in den Formulierungen ebenfalls in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können bei der Anwendung als Wachstumsregulatoren ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

In entsprechenden Aufwandmengen zeigen die erfindungsgemäßen Wirkstoffe darüberhinaus auch eine blattinsektizide Wirksamkeit.

Die Herstellung und die Verwendung der erfindungsgemäßen Wirkstoffe geht aus den nachfolgenden Beispielen hervor:

Herstellungsbeispiele:

Beispiel 1

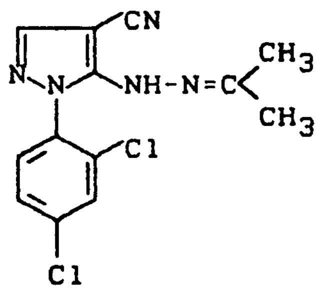

3 g (0,011 Mol) 4-Cyano-5-hydrazino-1-(2,4-dichlorphenyl)-pyrazol werden in 30 ml Aceton 2 Minuten zum Sieden erwärmt. Das Aceton wird abdestilliert und der kristalline Rückstand aus Toluol umkristallisiert.

Man erhält 2,7 g (78 % der Theorie) an 5-[N-(2-Propylidenimino)-amino]-4-cyano-1-(2,4-dichlorphenyl)-pyrazol vom Schmelzpunkt 175°C.

Herstellung der Ausgangsverbindungen

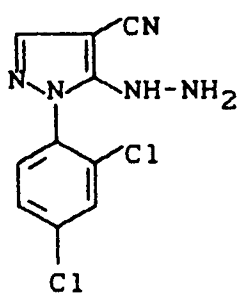

6,4 g (0,02 Mol) 5-Brom-4-cyano-1-(2,4-dichlorphenyl)-pyrazol und 15 g (0,2 Mol) Hydrazinhydrat in 80 ml Dioxan werden 20 Stunden bei Rückflußtemperatur gerührt, dann unter vermindertem Druck eingeengt, der Rückstand mit Wasser verrührt, abgesaugt und zweimal aus Ethanol umkristallisiert.

Man erhält 2,1 g (40 % der Theorie) an 4-Cyano-5-hydrazino-1-(2,4-dichlorphenyl)-pyrazol vom Schmelzpunkt 199-204°C.

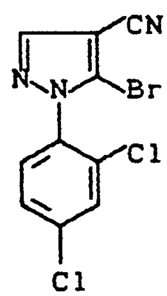

Zu einer Suspension aus 12,7 g (0,05 Mol) 5-Amino-4-cyano-1-(2,4-dichlorphenyl)-pyrazol in 100 ml Bromwasserstoffsäure gibt man bei -5°C bis 0°C 6 g (0,09 Mol) Natriumnitrit in 15 ml Wasser, rührt bis zum Ende der Gasentwicklung, wobei die Temperatur auf 30°C ansteigt. Der feste Rückstand wird abgesaugt, in Wasser aufgeschlämmt, mit Natriumhydrogencarbonat neutralisiert, wieder abgesaugt und getrocknet.

Man erhält 14,5 g (91,5 % der Theorie) an 5-Brom-4-cyano-1-(2,4-dichlorphenyl)-pyrazol vom Schmelzpunkt 84°C (Zers.).

Beispiel 2

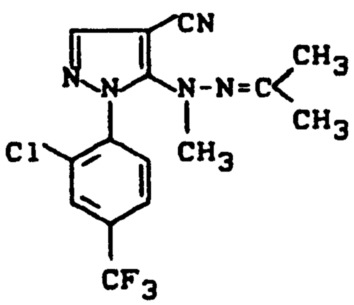

2,3 g (0,007 Mol) 5-(α-Methylhydrazino)-4-cyano-1-(2-chlor-4-trifluormethyl-phenyl)-pyrazol werden 2 Minuten lang in 20 ml Aceton zum Sieden erhitzt. Das Aceton wird abdestilliert und der ölige Rückstand säulenchromatographisch (Kieselgel/Laufmittel: Chloroform-Aceton 9:1) aufgetrennt.

Man erhält 2,0 g (76,4 % der Theorie) an 4-Cyano-5-[N-Methyl-N-(2-propylidenimino)-amino]-1-(2-chlor-4-trifluormethyl-phenyl)-pyrazol vom Schmelzpunkt 162°C.

Herstellung der Ausgangsverbindung

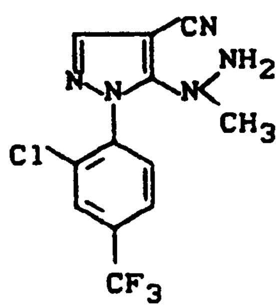

3,5 g (0,01 Mol) 5-Brom-4-cyano-1-(2-chlor-4-trifluormethyl-phenyl)-pyrazol und 13,8 g (0,3 Mol) N-Methylhydrazin werden in 100 ml Dioxan 5 Tage unter Rückfluß erhitzt, im Vakuum eingeengt und der Rückstand in Chloroform aufgenommen, dreimal mit Wasser gewaschen, über Natriumsulfat getrocknet und im Vakuum vom Lösungsmittel befreit.

Man erhält 2,3 g (72 % der Theorie) an 5-(α-Methylhydrazino)-4-cyano-1-(2-chlor-4-trifluormethyl-phenyl)-pyrazol als Öl.
$^1$H-NMR ($CDCl_3$/TMS als interner Standard) $\delta$ = 3,3 ppm (α-Methylhydrazinogruppe)

In entsprechender Weise und gemäß den allgemeinen Angaben zur Herstellung erhält man die folgenden 1-Aryl-pyrazole der allgemeinen Formel (I):

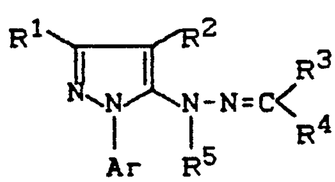

(I)

Tabelle 2

| Beispiel Nr. | $R^1$ | $R^2$ | $R^3$ | $R^4$ | $R^5$ | Ar | Schmelz-punkt/°C |
|---|---|---|---|---|---|---|---|
| 3 | H | $NO_2$ | $CH_3$ | $CH_3$ | H | 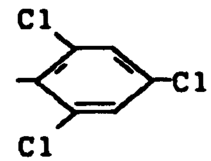 | 130-131 |
| 4 | H | CN | $CH_3$ | $CH_3$ | H | 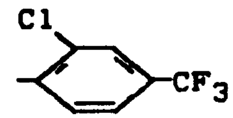 | 134-136 |
| 5 | H | $NO_2$ | $CH_3$ | $CH_3$ | H | 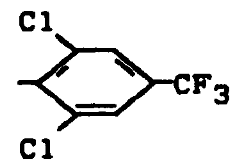 | 110-115 |
| 6 | H | $NO_2$ | $CH_3$ | $CH_3$ | H | 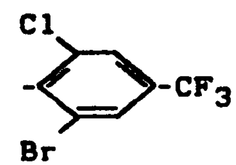 | 94-100 |

Anwendungsbeispiele:

In den folgenden Anwendungsbeispielen wurde die nachstehend aufgeführte Verbindung als Vergleichsubstanz eingesetzt:

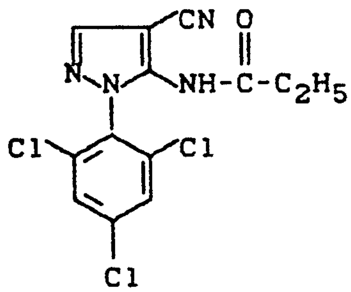

(A)

4-Cyano-5-propionamido-1-(2,4,6-trichlor-phenyl)-pyrazol (bekannt aus DE-OS 3 226 513)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Nutzpflanzenselektivität gegenüber dem Stand der Technik zeigt in diesem Test z.B die Verbindung gemäß dem Herstellungsbeispiel: 4.

Beispiel B

Wachstum bei Gerste

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und in Prozent des Zuwachses der Kontrollpflanzen berechnet. Es bedeuten 100 % einen Zuwachs wie bei den Kontrollen, Werte unter 100 % geben eine Wuchshemmung, Werte über 100 % eine Wuchsförderung wieder.

Eine deutliche Wirksamkeit im Vergleich zur unbehandelten Kontrolle zeigt in diesem Test z.B die Verbindung gemäß Herstellungsbeispiel 1.

0212354

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
++ starkes Austrocknen der Blätter, starker Blattfall
+++ sehr starkes Austrocknen der Blätter, sehr starker Blattfall

Eine deutliche Wirksamkeit im Vergleich zur unbehandelten Kontrolle zeigen in diesem Test beispielsweise die Verbindungen gemäß den Herstellungsbeispielen 1 und 4.

## Patentansprüche

1. 1-Aryl-pyrazole der allgemeinen Formel (I),

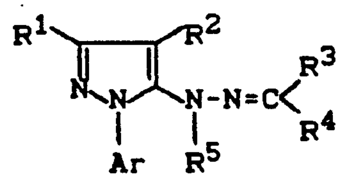

(I)

in welcher

$R^1$ für Wasserstoff oder Alkyl steht,

$R^2$ für Cyano oder Nitro steht,

$R^3$ und $R^4$ unabhängig voneinander jeweils für Wasserstoff, Alkyl, Alkoxyalkyl, Alkylthioalkyl, Halogenalkyl oder für gegebenenfalls substituiertes Aryl stehen, oder

$R^3$ und $R^4$ gemeinsam für einen zweifach ververknüpften Alkylenrest stehen,

$R^5$ für Wasserstoff oder Alkyl steht

und

Ar für gegebenenfalls substituiertes Phenyl oder für gegebenenfalls substituiertes Pyridyl steht.

2. 1-Aryl-pyrazole der Formel (I) gemäß Anspruch 1, in welcher

$R^1$ für Wasserstoff oder für geradkettiges oder verzweigtes Alkyl mit 1 bis 4 Kohlenstoffatomen steht,

$R^2$ für Cyano oder Nitro steht,

$R^3$ und $R^4$ unabhängig voneinander jeweils für Wasserstoff, für jeweils geradkettiges oder verzweigtes Alkyl, Alkoxyalkyl, Alkylthioalkyl oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen, oder für gegebenenfalls einfach oder mehrfach, gleich oder verschieden substituiertes Phenyl stehen, wobei als Substituenten jeweils in Frage kommen: Halogen, Cyano, Nitro oder jeweils geradkettiges oder verzweigtes Alkyl, Alkoxy, Alkylthio oder Halogenalkyl mit jeweils 1 bis 4 Kohlenstoffatomen und im Fall des Halogenalkyl mit 1 bis 9 gleichen oder verschiedenen Halogenatomen, oder

$R^3$ und $R^4$ gemeinsam für einen geradkettigen oder verzweigten, zweifach verknüpften Alkylenrest mit 3 bis 12 Kohlenstoffatomen stehen,

$R^5$ für Wasserstoff oder für geradkettiges oder verzweigtes Alkyl mit 1 bis 4 Kohlenstoffatomen steht und

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils in Frage kommen:
Cyano, Nitro, Halogen, jeweils geradkettiges oder verzweigtes Alkyl, Alkoxy oder Alkoxycarbonyl mit jeweils 1 bis 4 Kohlenstoffatomen im Alkylteil, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^6$

wobei

$R^6$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

m für eine Zahl 0, 1 oder 2 steht.

3. 1-Aryl-pyrazole der Formel (I) gemäß Anspruch 1, in welcher

$R^1$ für Wasserstoff, Methyl, Ethyl, n- oder i-Propyl, sowie n-, i-, s- oder t-Butyl steht,

$R^2$ für Cyano oder Nitro steht,

$R^3$ und $R^4$ unabhängig voneinander jeweils für Wasserstoff, Methyl, Ethyl, n- oder i-Propyl, n-, i-, s- oder t-Butyl, Methoxymethyl, Methylthiomethyl, Chlormethyl, Dichlormethyl, Trichlormethyl, Trifluormethyl, Chlorethyl, Bromethyl, Trichlorethyl, Trifluorethyl oder für gegebenenfalls ein- bis dreifach, gleich oder verschieden substituiertes Phenyl stehen, wobei als Substituenten jeweils in Frage kommen: Fluor, Chlor, Brom, Cyano, Nitro, Methyl, Ethyl, Methoxy, Methylthio oder Trifluormethyl, oder

$R^3$ und $R^4$ gemeinsam für einen geradkettigen, zweifach verknüpften Alkylenrest mit 4 bis 7 Kohlenstoffatomen stehen,

$R^5$ für Wasserstoff, Methyl oder Ethyl steht und

Ar für gegebenenfalls ein- bis fünffach, gleich oder verschieden substituiertes Phenyl oder für jeweils gegebenenfalls ein- bis vierfach,

gleich oder verschieden substituiertes 2-Pyridyl oder 4-Pyridyl steht, wobei als Substituenten jeweils in Frage kommen: Cyano, Nitro, Fluor, Chlor, Brom, Iod, Methyl, Ethyl, n- oder i-Propyl, n-, i-, s- oder t-Butyl, Methoxy, Ethoxy, Methoxycarbonyl, Ethoxycarbonyl, Trifluormethyl, Trichlormethyl, Difluorchlormethyl, Difluorchlorethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^6$, wobei

$R^6$ für Amino, Methylamino, Ethylamino, Dimethylamino, Diethylamino, Methyl, Ethyl, Fluordichlormethyl, Difluormethyl, Tetrafluorethyl, Trifluorchlorethyl oder Trifluormethyl steht und

m für eine Zahl 0, 1 oder 2 steht.

4. Verfahren zur Herstellung von 1-Aryl-pyrazolen der Formel (I)

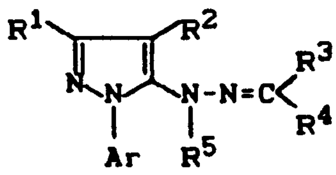

(I)

in welcher

$R^1$ für Wasserstoff oder Alkyl steht,

$R^2$ für Cyano oder Nitro steht,

$R^3$ und $R^4$ unabhängig voneinander jeweils für Wasserstoff, Alkyl, Alkoxyalkyl, Alkylthioalkyl, Halogenalkyl oder für gegebenenfalls substituiertes Arýl stehen, oder

$R^3$ und $R^4$ gemeinsam für einen zweifach ververknüpften Alkylenrest stehen,

$R^5$ für Wasserstoff oder Alkyl steht

und

Ar für gegebenenfalls substituiertes Phenyl oder für gegebenenfalls substituiertes Pyridyl steht,

dadurch gekennzeichnet, daß man

(a) 1-Aryl-5-hydrazino-pyrazole der Formel (II),

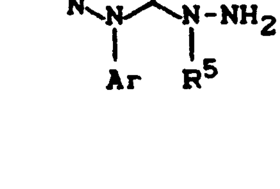

(II)

in welcher

$R^1$, $R^2$, $R^5$ und Ar die oben angegebene Bedeutung haben,

mit Aldehyden oder Ketonen der Formel (III),

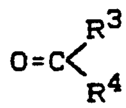

(III)

in welcher

$R^3$ und $R^4$ die oben angegebene Bedeutung haben,

gegebenenfalls in Gegenwart eines Verdünnungsmittels umsetzt, oder daß man

(b) die nach Verfahren (a) erhältlichen 1-Aryl-pyrazole der Formel (Ia),

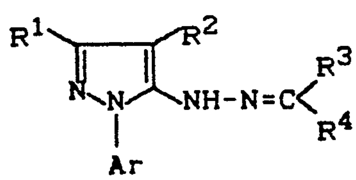

(Ia)

in welcher

$R^1$, $R^2$, $R^3$, $R^4$ und Ar die oben angegebene Bedeutung haben,

mit Alkylierungsmitteln der Formel (IV),

$$R^{5-1}-A \quad (IV)$$

in welcher

$R^{5-1}$ für Alkyl steht und

A für eine elektronenanziehende Abgangsgruppe steht,

gegebenenfalls in Gegenwart eines Verdünnungsmittels und gegebenenfalls in Gegenwart eines Säurebindemittels oder basischen Katalysators umsetzt.

5. Herbizide und pflanzenwuchsregulierende Mittel, gekennzeichnet durch einen Gehalt an mindestens einem 1-Aryl-pyrazol der Formel (I) gemäß den Ansprüchen 1 bis 4.

6. Verfahren zur Bekämpfung von Unkräutern, dadurch gekennzeichnet, daß man 1-Aryl-pyrazole der Formel (I) gemäß den Ansprüchen 1 bis 4 auf Unkräuter und/oder ihren Lebensraum einwirken läßt.

7. Verwendung von 1-Aryl-pyrazolen der Formel (I) gemäß den Ansprüchen 1 bis 4 zur Bekämpfung von Unkräutern und/oder als Pflanzenwachstumsregulatoren.

8. Verfahren zur Herstellung von herbiziden und/oder pflanzenwuchsregulierenden Mitteln, dadurch gekennzeichnet, daß man 1-Aryl-pyrazole der Formel (I) gemäß den Ansprüchen 1 bis 4 mit Streckmitteln und/oder oberflächenaktiven Substanzen vermischt.